# EUROPEAN PATENT APPLICATION

(11) **EP 4 733 403 A1**
(43) Date of publication of application: **29.04.2026**
(21) Application number: 24832252.1
(22) Date of filing: 17.05.2024
(51) Int. Cl.: C12P 13/00, C12P 7/40, C12N 15/77, C12N 9/88, C12P 7/46

(54) **PROCESS FOR PREPARING CADAVERINE DICARBOXYLATE**

(30) Priority: 28.06.2023 KR 20230083688; 31.07.2023 KR 20230099906
(71) Applicant: CJ Cheiljedang Corporation, Seoul 04560 (KR)
(72) Inventor: NOH, Seung-woo, Seoul 04560 (KR); LEE, Yong-Chan, Seoul 04560 (KR); PARK, Sang Min, Seoul 04560 (KR); LEE, Su Jin, Seoul 04560 (KR); KWAK, Dong Hun, Seoul 04560 (KR); LEE, Kang Hoon, Seoul 04560 (KR); SHIN, Ji Hyun, Seoul 04560 (KR); PARK, Jeong Ho, Seoul 04560 (KR); AHN, Sung Ahm, Seoul 04560 (KR)
(74) Representative: Meissner Bolte Partnerschaft mbB
(86) International application number: PCT/KR2024/006739
(87) International publication number: WO 2025/005468

(57) **Abstract**

The present disclosure relates to a method for preparing a cadaverine dicarboxylate (cadaverine succinate or cadaverine glutarate), which comprises a first step of obtaining a lysine dicarboxylate by culturing an L-lysine producing microorganism in a medium containing a dicarboxylate in the form of a diammonium dicarboxylate and a second step of converting the lysine dicarboxylate into a cadaverine dicarboxylate. According to the present disclosure, by performing the process according to the above-described procedure, a highly pure cadaverine dicarboxylate (cadaverine succinate or cadaverine glutarate) can be obtained without ion resin exchange, decarbonation, and distillation processes.

## Description

### [Technical Field]

The present disclosure relates to a technology for preparing a highly pure cadaverine dicarboxylate (cadaverine succinate or cadaverine glutarate) utilizing microbial fermentation and purification processes.

### [Background Art]

Cadaverine is a foul-smelling, toxic diamine compound produced by the putrefaction of animal tissue. Unlike hexanediamine, cadaverine is present in a liquid state at room temperature and more easily absorbs carbon dioxide, an acidic gas in the air, and this leads to a number of difficulties in transportation and storage of cadaverine. Therefore, salt-forming crystallization provides not only high-quality monomers but also easy transportation and storage.

Meanwhile, cadaverine succinate is a new type of bio-based nylon as the monomer component of nylon 54. This is widely applied in the fields of medicine, agriculture, and industry and is an environmentally friendly raw material. In addition, cadaverine glutarate is a raw material with high industrial utility as a precursor for the production of polyamide resin.

The cadaverine dicarboxylate preparation process generally comprises the following steps:
Synthesis of cadaverine: cadaverine synthesis involves fermentation of a proper microbial strain capable of producing lysine decarboxylase, an enzyme that catalyzes the decarboxylation of lysine to cadaverine.

Formation of cadaverine dicarboxylate: the formation of a cadaverine dicarboxylate involves the reaction of cadaverine with a dicarboxylic acid in the presence of a proper catalyst. The reaction typically involves mixing of the two monomers at a proper temperature and stirring for a certain period of time to ensure complete reaction.

Purification of cadaverine dicarboxylate: once the reaction is completed, the cadaverine dicarboxylate, a raw material is purified using various separation and purification techniques such as solvent extraction, chromatography, and crystallization.

According to the prior art, cadaverine succinate or cadaverine glutarate also has a high solubility in pure water, and the solubility remarkably increases as the temperature increases. The high solubility leads to a high viscosity of the solution and a low recovery rate, and this makes it difficult to crystallize cadaverine succinate in aqueous systems. In addition, there is a problem that unreacted succinic acid or unreacted glutaric acid is crystallized together and this decreases the purity of cadaverine succinate or cadaverine glutarate, the final product, and since this acid is required to be removed before crystallization, there are problems such as cost burden due to the additional process for removal of the acid, reduced process efficiency, and environmental pollution due to the use of chemical solvents.

### [Disclosure]

### [Technical Problem]

The problem to be solved by the present disclosure is to provide a method for preparing a cadaverine dicarboxylate (cadaverine succinate or cadaverine glutarate).

### [Technical Solution]

An object of the present disclosure is to provide a method of preparing cadaverine dicarboxylate, which comprises a first step of obtaining a lysine dicarboxylate by culturing an L-lysine producing microorganism in a medium containing a dicarboxate in the form of a diammonium dicarboxylate and a second step of converting the lysine dicarboxylate into a cadaverine dicarboxylate.

### [Advantageous Effects]

According to an aspect of the present disclosure, a lysine dicarboxylate is obtained by culturing an L-lysine producing microorganism in a medium containing a diammonium dicarboxylate and the lysine dicarboxylate is enzymatically converted into a cadaverine dicarboxylate, and thus the process is simplified as a separate process to remove a dicarboxylate is not required as well as a highly pure C4 or C5 cadaverine dicarboxylate can be prepared at a high yield as crystals of the dicarboxylic acid are not precipitated together as a by-product.

### [Brief Description of Drawings]

FIG. 1 is a flowchart illustrating a cadaverine dicarboxylate preparation process according to the present disclosure; and
FIG. 2 is a flowchart illustrating a cadaverine dicarboxylate preparation process according to an aspect of the present disclosure.

### [Detailed Description of the Invention]

Hereinafter, the configuration and effects of the present disclosure will be described in detail. Meanwhile, each description and each embodiment disclosed in the present disclosure may also be applied to other descriptions and other embodiments, respectively. In other words, all combinations of the various elements disclosed in the present disclosure fall within the scope of the present disclosure. In addition, the scope of the present disclosure is not limited to the specific description below.

An aspect of the present disclosure provides a method of preparing cadaverine dicarboxylate, which comprises a first step of obtaining a lysine dicarboxylate by culturing an L-lysine producing microorganism in a medium containing a dicarboxylate in the form of a diammonium dicarboxylate and a second step of converting the lysine dicarboxylate into a cadaverine dicarboxylate.

In the present disclosure, the term "dicarboxylic acid" refers to a dibasic acid containing two carboxyl groups, and is of high importance as a metabolic intermediate in the living body.

Specifically, the dicarboxylic acid may be an aliphatic dicarboxylic acid having 4 or 5 carbon atoms. In other words, in the present disclosure, the dicarboxylic acid refers to one aliphatic dicarboxylic acid selected from succinic acid or glutaric acid. In a case where the cadaverine dicarboxylate to be produced by the preparation method of the present disclosure is cadaverine succinate, it should be understood that all dicarboxylic acids added or introduced are succinic acid. In addition, in a case where the cadaverine dicarboxylate to be produced by the preparation method of the present disclosure is cadaverine glutarate, it should be understood that all dicarboxylic acids added or introduced are glutaric acid. As such, in the present disclosure, the dicarboxylic acid is understood to refer to one type of aliphatic dicarboxylic acid rather than a mixture of different types of dicarboxylic acids.

In the present disclosure, the term "succinic acid" is a dicarboxylic acid having 4 carbon atoms, has a chemical formula of C₄H₆O₄, and is generally present in the form of a sour, odorless white solid. Succinic acid is also called butanedioic acid or the like and is utilized as a precursor to a variety of industrial products, including polymers and plasticizers.

In the present disclosure, the term "glutaric acid" is a dicarboxylic acid having 5 carbon atoms, has a chemical formula of C₅H₈O₄, and is generally present in the form of a colorless crystal. The glutaric acid is also called 1,3-propanedicarboxylic acid, n-pyrotartaric acid, or the like, and is utilized as a precursor to a variety of industrial products, including polymers and plasticizers.

In the present disclosure, the term "cadaverine (CAD)" is a foul-smelling, toxic diamine compound, and is represented by a chemical formula of NH₂(CH₂)₅NH₂. Cadaverine may also be called 1,5-pentanediamine or pentamethylenediamine. In the present disclosure, cadaverine should be understood to include a cadaverine dicarboxylate form.

In the present disclosure, the term "cadaverine dicarboxylate" refers to a substance prepared by reacting the cadaverine in a diamine form with a dicarboxylic acid component, and has high industrial utility as a precursor for the preparation of polyamide resin. Specifically, in the present disclosure, the cadaverine dicarboxylate refers to one cadaverine dicarboxylate selected from cadaverine succinate or cadaverine glutarate.

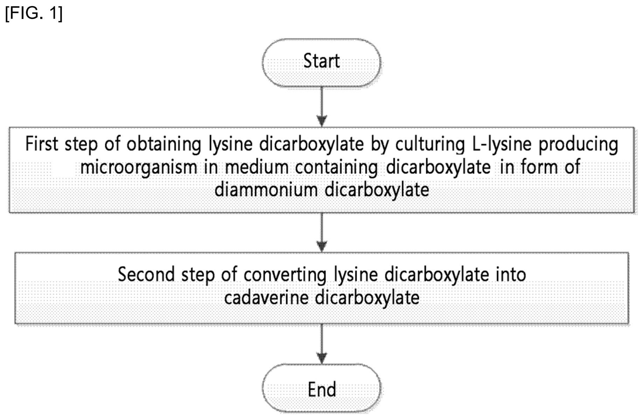

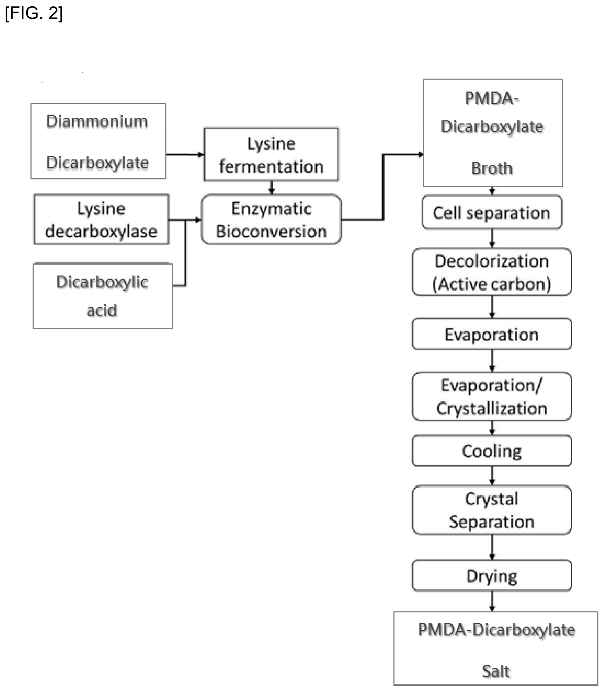

FIGS. 1 and 2 are flowcharts illustrating the cadaverine dicarboxylate preparation process according to an aspect of the present disclosure.

In the present disclosure, the term "L-lysine producing microorganism" includes both wild-type microorganisms and microorganisms that have undergone natural or artificial genetic modification, is a microorganism of which a specific mechanism is weakened or enhanced by a cause such as insertion of foreign genes or enhanced or inactivated activity of intrinsic genes, and may be a microorganism containing genetic modification for the production of L-lysine.

In an example, the L-lysine producing microorganism in the present disclosure may be a microorganism that naturally has the ability to produce L-lysine, a microorganism constructed by imparting the ability to produce L-lysine to a microorganism that does not have the ability to produce L-lysine, or a microorganism constructed by enhancing the ability to produce L-lysine of a microorganism having significantly low ability to produce L-lysine, but is not limited thereto. Specifically, in the present disclosure, the microorganism that produces L-lysine or the microorganism that has the ability to produce L-lysine may be a microorganism in which some genes in the L-lysine biosynthesis pathway are enhanced or weakened or some genes in the L-lysine degradation pathway are enhanced or weakened. The "enhancement" or "increase" in the ability to produce L-lysine means that the ability to produce L-lysine is improved compared to the parent strain or unmodified microorganism.

As a specific example, the microorganism may be a microorganism of the genus *Corynebacterium* or a microorganism of the genus *Escherichia.* The microorganism of the genus *Corynebacterium* may include all microorganisms belonging to the genus *Corynebacterium.* The microorganism of the genus *Corynebacterium may* specifically be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris* or *Corynebacterium flavescens,* and may more specifically be *Corynebacterium glutamicum,* but is not limited thereto. The microorganism of the genus *Escherichia* may include all microorganisms belonging to the genus *Escherichia.* The microorganism of the genus *Escherichia* may specifically be *Escherichia coli, Escherichia albertii, Escherichia blattae, Escherichia fergusonii, Escherichia hermannii* or *Escherichia vulneris,* and may more specifically be *Escherichia coli,* but is not limited thereto.

Culture of the L-lysine producing microorganism may be carried out according to appropriate media and culture conditions known in the art. This culture process may be easily adjusted and used by those skilled in the art depending on the selected microorganism. Specifically, the culture may be batch, continuous, or fed-batch culture, but is not limited thereto.

In the present disclosure, the term "medium" refers to a material in which nutrients necessary for culturing the microorganism are mixed as main components, and supplies water, nutrients and growth factors that are essential for survival and development. Specifically, as the medium used for culturing the L-lysine producing microorganism of the present disclosure, any medium used for culturing common microorganisms may be used without particular limitation as long as it contains a diammonium dicarboxylate as part or all of the nitrogen sources. The microorganism of the present disclosure may be cultured under aerobic conditions in a common medium containing nitrogen sources including a diammonium dicarboxylate, appropriate carbon sources, phosphorus sources, inorganic compounds, amino acids, vitamins and/or the like while the temperature, pH and the like are controlled.

The first step in the cadaverine dicarboxylate preparation method of the present disclosure is a step of obtaining a lysine dicarboxylate by culturing an L-lysine producing microorganism in a medium containing a dicarboxylate in the form of a diammonium dicarboxylate. This may be achieved through the fermentation process of a lysine producing microorganism.

In the first step, the dicarboxylate may be supplied to the medium in the form of a diammonium dicarboxylate, and thus the dicarboxylate in the present disclosure should be understood to include a diammonium dicarboxylate form.

In the present disclosure, the term "diammonium dicarboxylate" is a form in which two hydrogen atoms of a dicarboxylic acid are substituted with ammonium, and is used as a main nitrogen source in the medium. The diammonium dicarboxylate may be prepared by gently mixing a dicarboxylic acid with ammonia water to have a neutral pH and then concentrated to be used in the form of crystals or liquid form, but is not limited thereto.

In the preparation method of the present disclosure, by introducing 81 mol% or more of dicarboxylate supplied throughout the process into the medium in the form of a diammonium dicarboxylate in the first step, a highly pure cadaverine dicarboxylate can be prepared as well as the process is simplified and the side effect that a dicarboxylate is precipitated together with a cadaverine dicarboxylate can be reduced.

Specifically, the diammonium dicarboxylate in the first step may be contained in the medium at a high ratio, specifically at 61 mol% or more with respect to the dicarboxylate supplied throughout the process of the preparation method of the present disclosure.

More specifically, the diammonium dicarboxylate in the first step may be contained to be 61 mol% or more, 68 mol% or more, 73 mol% or more, 81 mol% or more, 82 mol% or more, 83 mol% or more, 84 mol% or more, 85 mol% or more, 86 mol% or more, 87 mol% or more, 88 mol% or more, 89 mol% or more, 90 mol% or more, 91 mol% or more, 92 mol% or more, 93 mol% or more, 94 mol% or more, 95 mol% or more, 96 mol% or more, 97 mol% or more, 98 mol% or more, 99 mol% or more, or 100 mol% of the dicarboxylate supplied throughout the process of the preparation method of the present disclosure.

In the preparation method of the present disclosure, by containing a large amount of diammonium dicarboxylate in the medium, it is possible to provide the advantage of preparing a highly pure cadaverine dicarboxylate without performing a separate dicarboxylate removing step such as ion resin exchange, decarbonation, or distillation process unlike a conventional process in which ammonium sulfate or the like is introduced. By using a diammonium dicarboxylate, it is possible to easily adjust the molar ratio of dicarboxylate added throughout the process/cadaverine in the process liquid after the second step.

Specifically, the diammonium dicarboxylate in the first step may be contained so that the molar ratio of the diammonium dicarboxylate to lysine to be produced(diammonium dicarboxylate/lysine) is 0.63 to 1. More specifically, the diammonium dicarboxylate in the first step may be contained so that the molar ratio of the diammonium dicarboxylate to lysine to be produced(diammonium dicarboxylate/lysine) is 0.6 to 0.98, 0.73 to 0.98, 0.78 to 0.98, or 0.8 to 0.98, or 0.83 to 0.98, still more specifically 0.85 to 0.98. At this time, the amount of lysine to be produced should be understood to refer to the total amount including the amount of lysine in a lysine dicarboxylate form.

As an example, the diammonium dicarboxylate in the first step may be contained so that the molar ratio of the diammonium dicarboxylate to lysine to be produced(diammonium dicarboxylate/lysine) is in a range consisting of one lower limit selected from 0.63, 0.65, 0.7, 0.73, 0.78, 0.8, 0.85, 0.86, 0.87, 0.88, or 0.89 and/or one upper limit selected from 1, 0.99, 0.98, 0.97, 0.96, 0.95, 0.94, 0.93, or 0.92.

Specifically, the diammonium dicarboxylate in the first step may be contained at 0.4 mol/L to 0.8 mol/L based on the L-lysine producing microorganism fermentation broth. More specifically, the diammonium dicarboxylate in the first step may be contained at 0.48 mol/L to 0.7 mol/L, 0.5 mol/L to 0.7 mol/L, 0.55 mol/L to 0.7 mol/L, or 0.55 mol/L to 0.65 mol/L based on the L-lysine producing microorganism fermentation broth. By adding the diammonium dicarboxylate at the above concentration, the ammonium ion may act as a nitrogen source during fermentation and the dicarboxylate and lysine may form a salt to be contained in the fermentation broth in a dissolved state.

A lysine dicarboxylate may be produced through the fermentation of an L-lysine producing microorganism in a medium containing a diammonium dicarboxylate. The lysine dicarboxylate can be introduced into the subsequent processes as it is, in the form of a fermentation broth containing bacteria, in the form of a fermentation broth from which the bacteria have been removed, or after purification.

The concentration of lysine in the fermentation broth in which a lysine producing microorganism has been cultured in the first step of the present disclosure is not limited, but may be 90 g/L to 300 g/L, 92 g/L to 250 g/L, 92 g/L to 160 g/L, or 92 g/L to 100 g/L based on the L-lysine producing microorganism fermentation broth.

The carbon source of the medium may include carbohydrates such as glucose, fructose, sucrose, maltose, and isomers thereof; sugar alcohols such as mannitol and sorbitol; organic acids such as pyruvic acid, lactic acid, and citric acid; and amino acids such as glutamic acid, methionine, and lysine. Natural organic nutrient sources such as starch hydrolysates, molasses, blackstrap molasses, rice bran, cassava, sugarcane bagasse and corn steep liquor may be used, specifically, carbohydrates such as glucose and sterilized and pre-treated molasses (that is, molasses converted into reducing sugars) may be used, and appropriate amounts of other carbon sources may be used variously without limitation. These carbon sources may be used singly or in combination of two or more kinds thereof, but are not limited thereto.

As a specific example, the carbon source that may be contained in the culture medium of the present disclosure may include glucose, maltose, or maltose isomers. More specifically, the carbon source may include one or more selected from glucose, maltose or maltose isomers (isomaltose) or a combination of two or more thereof, but is not limited thereto.

The phosphorus source of the medium may include potassium phosphate monobasic and potassium phosphate dibasic or sodium-containing salts corresponding to these. As the inorganic compounds, sodium chloride, calcium chloride, iron chloride, magnesium sulfate, iron sulfate, manganese sulfate, calcium carbonate and the like may be used.

The culture medium may contain metal salts such as magnesium sulfate or iron sulfate necessary for growth. Finally, in addition to the substances, substances essential for growth such as amino acids and vitamins may be used. Precursors suitable for the culture medium may be used. The above-mentioned raw materials may be added to the culture solution in an appropriate manner, for example, batchwise or continuously during the culture process, but are not limited thereto.

The nitrogen source of the medium may include a diammonium dicarboxylate. As a nitrogen source, common media for culturing microorganisms may contain inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and defatted soybean cake or decomposition products thereof, but a diammonium dicarboxylate may be contained as the only nitrogen source or as an additional nitrogen source in an aspect of the present disclosure.

After the first step of producing a lysine dicarboxylate by culturing an L-lysine producing microorganism in a medium containing a diammonium dicarboxylate, a second step of converting the lysine dicarboxylate into a cadaverine dicarboxylate may be performed.

The second step refers to a step of converting a lysine dicarboxylate into a cadaverine dicarboxylate (cadaverine succinate or cadaverine glutarate), the desired product.

In a specific example, the second step may comprise additionally adding a dicarboxylic acid to the process liquid. The process liquid may be a fermentation process liquid obtained in the first step.

The dicarboxylic acid added in the second step may not be added or may be added in a trace amount of 0.45 mol/L or less, 0.31 mol/L or less, 0.18 mol/L or less, 0.15 mol/L or less, 0.12 mol/L or less, 0.1 mol/L or less, 0.08 mol/L or less, 0.05 mol/L or less, 0.04 mol/L or less, 0.03 mol/L or less, 0.02 mol/L or less, or 0.01 mol/L or less based on the volume of the process liquid, specifically the volume of the fermentation process liquid obtained in the first step.

In a specific example, in a case where the dicarboxylic acid is succinic acid, the dicarboxylic acid added in the second step may not be added or may be added in a trace amount of 0.15 mol/L or less.

In a specific example, in a case where the dicarboxylic acid is glutaric acid, the dicarboxylic acid added in the second step may not be added or may be added in a trace amount of 0.08 mol/L or less.

In a case where a dicarboxylic acid is additionally added to the process liquid in the second step, the dicarboxylic acid may be added before, after, or simultaneously with the conversion reaction, but is not limited thereto.

In a case where a dicarboxylic acid is additionally added in the second step, the amount of dicarboxylate added throughout the process of the preparation method of the present disclosure is understood to be the sum of the amount of diammonium dicarboxylate added to the medium in the first step and the amount of dicarboxylic acid added in the second step. In a case where a dicarboxylic acid is not additionally added to the process liquid in the second step, the amount of dicarboxylate added throughout the process of the preparation method of the present disclosure is understood to be the same as the amount of diammonium dicarboxylate added to the medium in the first step.

Specifically, the amount of dicarboxylate added in the preparation method of the present disclosure may be determined considering the amount of cadaverine in the process liquid to be obtained after the second step. Specifically, the amount of dicarboxylate added in the preparation method of the present disclosure with respect to the amount of cadaverine in the process solution after the second step may be 0.85 to 1.10 in terms of molar ratio.

More specifically, the amount of dicarboxylate added in the preparation method of the present disclosure with respect to the amount of cadaverine in the process solution after the second step may be 0.85 to 1.07, 0.87 to 1.06, 0.88 to 1.05, 0.89 to 1.03, 0.88 to 1.02, 0.89 to 1.0, 0.9 to 1.10, 0.93 to 1.08, 0.93 to 1.07, 0.95 to 1.06 or 0.95 to 1.05 in terms of molar ratio.

As an example, the amount of dicarboxylate added in the preparation method of the present disclosure with respect to the amount of cadaverine in the process solution after the second step may be in a range consisting of one lower limit selected from 0.83, 0.84, 0.85, 0.86, 0.87, 0.88, 0.89, 0.9, 0.91, 0.92, 0.93, 0.94, or 0.95 and/or one upper limit selected from 1.10, 1.09, 1.08, 1.07, 1.06, 1.05, 1.04, 1.03, 1.02, 1.01, or 1.0 in terms of molar ratio.

In a specific example, in a case where the dicarboxylate is succinate, the amount of dicarboxylate added in the preparation method of the present disclosure with respect to the amount of cadaverine in the process solution after the second step may be 0.9 to 1.10, 0.91 to 1.09, 0.92 to 1.08, 0.93 to 1.07, 0.94 to 1.06, or 0.95 to 1.05 in terms of molar ratio.

In the preparation method of the present disclosure, when succinate is added at a molar ratio in the above range with respect to the amount of cadaverine calculated to be obtained after the second step, one technical significance exists in the finding that succinic acid is not precipitated as crystals together with cadaverine succinate and it is thus possible to produce highly pure cadaverine succinate at a high yield.

By adjusting the molar ratio of succinate added to cadaverine to be obtained, it is possible to maintain a purity of 99% of cadaverine succinate up to the 4th circulation when the mother liquor is circulated, as well as cadaverine succinate can be obtained at a total yield of 30% or more, 32% or more, 51% or more, 53% or more, 65% or more, 68% or more, 75% or more, 78% or more, 80% or more, 81% or more, 82% or more, 83% or more, 84% or more, or 85% or more depending on the number of mother liquor circulations.

In a specific example, in a case where the dicarboxylate is glutarate, the amount of dicarboxylate added in the preparation method of the present disclosure with respect to the amount of cadaverine in the process solution after the second step may be 0.8 to 1.05, 0.83 to 1.04, 0.85 to 1.03, 0.87 to 1.02, 0.88 to 1.01, or 0.89 to 1.0 in terms of molar ratio.

In the preparation method of the present disclosure, when glutarate is added at a molar ratio in the above range with respect to the amount of cadaverine calculated to be obtained after the second step, one technical significance exists in the finding that glutaric acid is not precipitated as crystals together with cadaverine glutarate and it is thus possible to produce highly pure cadaverine glutarate at a high yield.

By adjusting the molar ratio of glutarate added to cadaverine to be obtained, it is possible to maintain a purity of 99% of cadaverine glutarate up to the 3rd circulation when the mother liquor is circulated, and cadaverine glutarate can be obtained at a total yield of 49% or more, 50% or more, 52% or more, 74% or more, 75% or more, 77% or more, 86% or more, 87% or more, 88% or more, 92% or more, 93% or more, or 94% depending on the number of mother liquor circulations.

In the second step, the conversion reaction of a lysine dicarboxylate into a cadaverine dicarboxylate may be an enzymatic conversion reaction.

As a specific example, the lysine dicarboxylate may comprise both a lysine dicarboxylate obtained in the first step and a lysine dicarboxylate produced in the second step in a case where a dicarboxylic acid is additionally introduced in the second step.

Specifically, the enzymatic conversion reaction in the second step may be conducted using a protein having lysine decarboxylase activity or a microorganism expressing a protein having the activity.

As one specific example, a protein having lysine decarboxylase activity or a seed culture solution of a microorganism expressing a protein having the activity may be introduced into the fermentation process liquid in the first step, but is not limited thereto.

In the present disclosure, the term "decarboxylase" is an enzyme that catalyzes the production of carbon dioxide by eliminating the carboxyl group, an organic acid, and may also be referred to as carboxy-lyase, and carbon-carbon lyase etc.

The protein is not particularly limited as long as it exhibits lysine decarboxylase activity, but may be, for example, *Pseudomonas thermotolerans* -derived PtLDC protein or *Escherichia* coli-derived CadA protein, but the protein sequence may be obtained using GenBank, a known database, the protein may be expressed using the microorganism, or a commercially available enzyme may be purchased and used, but the protein is not limited thereto.

The microorganism expressing the protein may be a microorganism transformed to express the protein. The transformed microorganism is not limited to prokaryotic microorganisms and eukaryotic microorganisms as long as it is transformed to express a protein having decarboxylase activity. The microorganism transformed to express a protein having decarboxylase activity can convert a lysine dicarboxylate in the process liquid into a cadaverine dicarboxylate by releasing the protein having enzyme activity into the seed culture solution.

Specific examples of the microorganism may include the genus *Escherichia,* the genus *Erwinia,* the genus *Serratia,* the genus *Providencia,* and coryneform microbial strains. The microorganism may be specifically a microorganism belonging to the genus *Escherichia* or the genus *Corynebacterium,* more specifically *Escherichia coli* or *Corynebacterium glutamicum,* but is not limited thereto.

The conversion reaction in the second step may be conducted for 20 minutes to 3 hours, more specifically for 0.5 to 1.5 hours, for example, for 1 hour, but is not limited thereto.

The conversion reaction in the second step may be conducted at 30°C to 60°C, more specifically at 40°C to 50°C, for example, at 45°C, but is not limited thereto.

The conversion reaction in the second step may be conducted at a pH of 7.5 to 9, more specifically at a pH of 7.8 to 8.7, still more specifically at a pH of 8 to 8.5. In an embodiment of the second step, the pH may be adjusted to the above range by the dicarboxylic acid selectively and additionally introduced, and in another embodiment, the pH may be adjusted to the above range by the addition of CO₂. When the pH range for the conversion reaction in the second step is adjusted to the above range, the conversion rate of a lysine dicarboxylate to a cadaverine dicarboxylate may be significantly increased.

The method of preparing cadaverine dicarboxylate preparation method of the present disclosure may further comprise a recovery step of recovering the obtained cadaverine dicarboxylate after the second step.

The recovery may be to collect a cadaverine dicarboxylate by a suitable method known in the art.

For example, a cadaverine dicarboxylate can be collected by centrifugation, filtration, concentration, crystallization, extraction, ultrasonic disruption, ultrafiltration, dialysis, various kinds of chromatography such as molecular sieve chromatography (gel filtration), adsorption chromatography, ion exchange chromatography, and affinity chromatography, HPLC, or a combination of these methods.

In a specific example of the present disclosure, the recovery step may comprise one or more of a filtration step, a concentration step, or a crystallization step.

The filtration step refers to a step of removing impurities from the conversion liquid containing a cadaverine dicarboxylate obtained in the second step. As an example, the filtration may comprise removing bacteria in the cadaverine dicarboxylate process liquid by performing membrane filtration or the like, or performing activated carbon filtration or the like.

The concentration step refers to a step of performing concentration to increase the solid ratio in the conversion liquid, and may be performed after the filtration step. As an example, the concentration may be conducted under reduced pressure using a rotary evaporator.

Specifically, the concentration step may be to perform concentration so that the solid content becomes 70% to 85% (w/w), more specifically 73% to 85% (w/w), 75% to 82% (w/w), 70% to 80% (w/w), 75% to 85% (w/w), 74% to 83% (w/w) w), 78% to 82%(w/w), or 73% to 77%(w/w), for example, 75% (w/w). When concentration in the concentration step is performed so that the solid content is in the above range, the recovery rate and purity of cadaverine dicarboxylate can be maintained excellently.

The concentration step may be performed at 45°C to 65°C, but is not limited thereto.

The crystallization step may be a cooling crystallization step, and this refers to a step of cooling the conversion liquid and precipitating the conversion liquid into crystals to obtain cadaverine dicarboxylate crystals, the finally desired product.

Specifically, the cooling may be cooling the conversion liquid to 20°C to 30°C, more specifically to 23°C to 27°C, for example, to 25°C, but is not limited thereto.

Specifically, the cooling rate may be 5°C to 20°C/hour, more specifically 5°C to 15°C/hour, for example, 10°C/hour, but is not limited thereto.

In an embodiment, the recovery step may further comprise a stirring step of stirring the process liquid. The stirring step may be performed simultaneously with the cooling crystallization step, in the middle of the cooling crystallization step, before the cooling crystallization step, or after the cooling crystallization step. Through this stirring step, time to sufficiently stir the process liquid may be provided and crystal precipitation may be facilitated.

In an embodiment, the recovery step may further comprise a mother liquor circulation step of recirculating the mother liquor containing a cadaverine dicarboxylate that has not been crystallized to the feed liquid for the concentration step after the crystallization step.

Specifically, in the mother liquor circulation step, the number of mother liquor circulations may be 0 to 3 times, 1 to 3 times, 1 to 4 times, 1 to 5 times, 1 to 10 times, or more times. At this time, the number of mother liquor circulations of 0 means that the mother liquor circulation step is not performed.

Hitherto, in the step of crystallizing a cadaverine dicarboxylate, a relatively large amount of cadaverine dicarboxylate has still remained in the mother liquor after crystallization, and there has been a problem that the yield is as low as less than 50%. In the present disclosure, as a method for improving the yield of cadaverine dicarboxylate, the mother liquor in which a cadaverine dicarboxylate remains is recirculated to the feed liquid for the concentration step 1 to 3 times, 1 to 4 times, 1 to 5 times, 1 to 10 times, or more times, and thus the total yield of cadaverine dicarboxylate can be increased.

In an embodiment, the recovery step may further comprise a step of washing, separating, and drying the crystallized process liquid.

In the drying step, the moisture contained in the crystals may be removed through drying, and a highly pure cadaverine dicarboxylate may be commercialized. After drying, cadaverine dicarboxylate crystals may be provided in a powder form, but the formulation may vary as needed.

In an embodiment, the recovery step may further comprise a decolorization step. The decolorization may be performed using activated carbon, anion resin or the like, but is not limited thereto.

The cadaverine dicarboxylatepreparation methodof the present disclosure may comprise an additional purification step. The purification may be performed by a suitable method known in the art. In an example, in a case where the cadaverine dicarboxylate preparation method of the present disclosure comprises both a recovery step and a purification step, the recovery step and the purification step may be performed continuously or discontinuously regardless of the order, or may be performed simultaneously or by being integrated into one step, but are not limited thereto.

The preparation method of the present disclosure may not comprise a separate process for removing a dicarboxylate in the recovery step. In the case of a conventional cadaverine dicarboxylate production process, a dicarboxylate is precipitated together as crystals during the crystallization process to decrease the purity, and in order to prevent this, a separate process to remove the dicarboxylate before crystallization is required. However, the preparation method of the present disclosure provides the technical advantage that a highly pure cadaverine dicarboxylate can be prepared without performing a separate dicarboxylate removing process.

In an embodiment, in a case where the dicarboxylate is succinate, the method of the present disclosure can afford cadaverine succinate having a purity of 99% or more in 0 to 4 times of mother liquor circulation. In an embodiment, in a case where the dicarboxylate is succinate, based on four times of mother liquor circulation, the cumulative total yield of cadaverine succinate may be more than 80%, more specifically 81% or more, 82% or more, 83% or more, or 84% or more.

In an embodiment, in a case where the dicarboxylate is glutarate, the method of the present disclosure can afford cadaverine glutarate having a purity of 99% or more in 0 to 3 times of mother liquor circulation. In an embodiment, in a case where the dicarboxylate is glutarate, based on three times of mother liquor circulation, the cumulative total yield of cadaverine glutarate may be more than 90%, more specifically 92% or more, 93% or more, or 94% or more.

Through the method of the present disclosure, a highly pure cadaverine dicarboxylate can be produced without the decarbonation and distillation processes required at the time of conventional cadaverine liquid production.

### [Modes for Carrying out the Invention]

Hereinafter, the present disclosure will be described in more detail with reference to Examples. However, the following Examples are only preferred embodiments for illustrating the present disclosure and are therefore not intended to limit the scope of the present disclosure thereto. Meanwhile, technical matters not described in this specification can be fully understood and easily implemented by those skilled in the technical field of the present disclosure or similar technical fields.

In the above, the preparation process of a cadaverine dicarboxylate (cadaverine succinate or cadaverine glutarate) according to an aspect of the present disclosure has been explained. Hereinafter, the advantageous effects mentioned in the present disclosure will be explained through the experimental results of Examples and Comparative Examples of each of cadaverine succinate and cadaverine glutarate.

### Preparation Example 1. Preparation of cadaverine succinate

### Preparation Example 1-1. Lysine succinate production and fermentation process

Seeds of *Corynebacterium glutamicum* strain (KCCM12154P, US 2021-0355514 A1) having the ability to produce lysine were obtained through solid phase culture and flask culture, seed culture was performed in a fermenter, and then the main production and fermentation were performed. Fermentation was carried out through fermenter culture at 36°C for 30 hours at 900 rpm.

For the production of lysine succinate using *Corynebacterium,* diammonium succinate was supplied at a level of 76 to 99 g/L to replace the existing ammonium sulfate, and fermentation was performed to obtain lysine succinate at a level of 132 to 172 g/L and lysine at a level of 99 g/L. At this time, in Examples, the molar ratio of succinate/lysine in the fermentation process liquid was adjusted to a level of 0.74 to 0.96.

### Preparation Example 1-2. Conversion reaction of lysine succinate into cadaverine succinate

For the conversion reaction, *Escherichia coli* (US 2018-0030430 A1) in which PtLDC enzyme, a lysine decarboxylase gene derived from *Pseudomonas thermotolerans,* was overexpressed with the pET-Deut1 vector was used.

The enzyme conversion liquid obtained by seed culture with the enzyme strain was supplied to the fermentation process liquid of lysine succinate prepared in Preparation Example 1-1 at a level of 10% by mass to conduct the conversion reaction. The conversion reaction was conducted for approximately 1 hour while the temperature was maintained at 45°C to 50°C. The pH was adjusted to 8.0 to 8.5, and for this, CO₂ was introduced for neutralization in Examples 1-1 to 1-3 below and a dicarboxylic acid was additionally introduced at a level of 0.03 mol/L (based on the volume of the fermentation process liquid of lysine succinate) in Examples 1-1 and 1-3. As a result, it was found that the conversion rate into cadaverine succinate was 97% to 98%. Specifically, after the conversion reaction, cadaverine succinate was obtained at a level of 117 to 143 g/L (based on the volume of the fermentation process liquid of lysine succinate) and cadaverine was obtained at a level of 68 g/L (based on the volume of the fermentation process liquid of lysine succinate). Subsequently, in Examples 1-4 to 1-7, succinic acid was additionally introduced at a level of 0.02 to 0.15 mol/L to prepare a process liquid.

### Preparation Example 1-3. Subsequent processes after obtaining of cadaverine succinate

The following subsequent processes were performed on the process liquid containing cadaverine succinate obtained in Preparation Example 1-2 to obtain cadaverine succinate in a crystal form.

### [Bacteria removing step]

Bacteria in the cadaverine succinate process liquid were removed by performing membrane filtration using a membrane having a size of 0.1 µm.

### [Step of removing impurities using activated carbon]

Activated carbon was added at a level of 10% based on the weight of cadaverine succinate in the filtrate from which the bacteria had been separated. The process liquid to which activated carbon had been added was heated to 60°C and stirred for 1 hour to decolorize, and then the activated carbon was filtered through filter paper.

### [Step of concentrating cadaverine succinate process liquid from which impurities had been removed]

The liquid filtered above was concentrated under reduced pressure in a rotary evaporator at about 45°C to 65°C and 40 to 70 torr until the solid content in the filtrate became 75% to 82% by weight.

### [Steps of cooling and crystallizing, separating and then drying cadaverine succinate concentrate]

The concentrate was cooled from 45°C to 35°C to 25°C at a rate of 5°C/hr. The precipitated crystals were separated from the mother liquor using a centrifuge. The crystals were washed with 10% water based on the weight of cadaverine succinate. The separated crystals were dried for one day, and then the purity thereof was measured by HPLC.

### [Step of recirculating mother liquor to crystallization step]

The mother liquor separated above was recirculated to the [step of concentrating cadaverine succinate process liquid from which impurities had been removed].

### Experimental Example 1. Examination of changes in purity and yield of cadaverine succinate depending on molar ratio of succinate to cadaverine

In Experimental Example 1, the method according to Preparation Examples 1-1 to 1-3 was used, but it was examined how the purity and yield of cadaverine succinate change depending on the molar ratio of succinate added throughout the process to cadaverine in the process liquid after the second step.

### Example 1-1: Case where molar ratio of succinate added/cadaverine in process liquid after second step is 0.8

A liquid for conversion into cadaverine succinate having a molar ratio of succinate /cadaverine of 0.8 was supplied by 1,000 ml and allowed to pass through a membrane having a size of 0.1 µm to remove microorganisms. The liquid for conversion into cadaverine succinate having a molar ratio of succinate /cadaverine of 0.8 was prepared according to the preparation method of Preparation Example 1-1 and Preparation Example 1-2 but the sum of the amount of diammonium succinate introduced in the fermentation process to produce lysine in Preparation Example 1-1. and the amount of succinic acid introduced in the reaction for conversion into cadaverine succinate (limited to Examples in which succinic acid was additionally introduced in the second step) in Preparation Example 1-2. was controlled to be 0.8 of the number of moles of cadaverine that had been converted.

The filtrate was decolorized using activated carbon, filtered through filter paper, then concentrated under reduced pressure at 40 torr, and concentrated so that the solid content became a level of 78% by weight. The concentrate was cooled and crystallized from 35°C to 25°C. The crystals and mother liquor were separated from each other through centrifugation, the separated crystals were dried for one day, and the purity thereof was then measured by HPLC. The mother liquor in which a succinate remained was recirculated to the cadaverine succinate feed liquid having a molar ratio of succinate /cadaverine of 0.8, and then the concentration step was performed again. Recirculation of the mother liquor was carried out sequentially.

### Example 1-2: Case where molar ratio of succinate added/cadaverine in process liquid after second step is 0.9

A liquid for conversion into cadaverine succinate having a molar ratio of succinate /cadaverine of 0.9 was supplied, and the purification process was performed while the mother liquor was sequentially recirculated in the same manner as in Example 1-1.

### Example 1-3: Case where molar ratio of succinate added/cadaverine in process liquid after second step is 0.95

A liquid for conversion into cadaverine succinate having a molar ratio of succinate /cadaverine of 0.95 was supplied, and the purification process was performed while the mother liquor was sequentially recirculated in the same manner as in Example 1-1.

### Example 1-4: Case where molar ratio of succinate added/cadaverine in process liquid after second step is 1.01

A liquid for conversion into cadaverine succinate having a molar ratio of succinate /cadaverine of 1.01 was supplied, and the purification process was performed while the mother liquor was sequentially recirculated in the same manner as in Example 1-1.

### Example 1-5: Case where molar ratio of succinate added/cadaverine in process liquid after second step is 1.05

A liquid for conversion into cadaverine succinate having a molar ratio of succinate /cadaverine of 1.05 was supplied, and the purification process was performed while the mother liquor was sequentially recirculated in the same manner as in Example 1-1.

In Examples 1-3 to 1-5, the molar ratio of succinate /lysine in the lysine fermentation step was adjusted to be 0.88 to 0.96.

### Example 1-6: Case where molar ratio of succinate added/cadaverine in process liquid after second step is 1.10

A liquid for conversion into cadaverine succinate having a molar ratio of succinate /cadaverine of 1.10 was supplied, and the purification process was performed while the mother liquor was sequentially recirculated in the same manner as in Example 1-1.

### Example 1-7: Case where molar ratio of succinate added/cadaverine in process liquid after second step is 1.20

A liquid for conversion into cadaverine succinate having a molar ratio of succinate /cadaverine of 1.20 was supplied, and the purification process was performed while the mother liquor was sequentially recirculated in the same manner as in Example 1-1.

The measured physical properties of the process liquids and cadaverine succinate prepared according to Examples 1-1 to 1-7 described above are as shown in Tables 1 to 5 below.

Table 1 is for the process liquids to which the mother liquor is not circulated.

**[Table 1]**

| **No mother liquor circulation** | **Exampl e 1-1** | **Exampl e 1-2** | **Exampl e 1-3** | **Exampl e 1-4** | **Exampl e 1-5** | **Exampl e 1-6** | **Exampl e 1-7** |
|---|---|---|---|---|---|---|---|
| **Molar ratio of succinate /cadaverine** | 0.8 | 0.9 | 0.95 | 1.01 | 1.05 | 1.1 | 1.2 |

| **Feed liquid for crystallization** | | | | | | | |
|---|---|---|---|---|---|---|---|
| **Fresh feed liquid, ml** | 1000 | 1000 | 1000 | 1000 | 1000 | 1000 | 1000 |
| **Amount of cadaverine in crystallization liquid, g** | 362 | 362 | 362 | 362 | 362 | 362 | 362 |
| **Amount of cadaverine in crystal produced, g** | 87 | 103 | 110 | 116 | 109 | 103 | 89 |
| **Amount of cadaverine in mother liquor, g** | 275 | 259 | 252 | 246 | 253 | 259 | 273 |

| **Results** | | | | | | | |
|---|---|---|---|---|---|---|---|
| **Purity, %** | 99.0 | 99.0 | 99.0 | 99.0 | 99.0 | 99.0 | 88.5 |
| **Yield, %** | 24.1 | 28.5 | 30.3 | 32.0 | 30.2 | 28.4 | 24.7 |
| **Total yield,** % | 24.1 | 28.5 | 30.3 | 32.0 | 30.2 | 28.4 | 24.7 |

Table 2 is for the process liquids to which the mother liquor was circulated one time.

**[Table 2]**

| **One time of mother liquor circulation** | **Exampl e 1-1** | **Exampl e 1-2** | **Exampl e 1-3** | **Exampl e 1-4** | **Exampl e 1-5** | **Exampl e 1-6** | **Exampl e 1-7** |
|---|---|---|---|---|---|---|---|
| **Molar ratio of succinate /cadaverine** | 0.8 | 0.9 | 0.95 | 1.01 | 1.05 | 1.1 | 1.2 |

| **Feed liquid for crystallization** | | | | | | | |
|---|---|---|---|---|---|---|---|
| **Fresh feed liquid, ml** | 1000 | 1000 | 1000 | 1000 | 1000 | 1000 | 1000 |
| **Amount of cadaverine in crystallization liquid, 9** | 637 | 621 | 614 | 608 | 615 | 621 | 635 |
| **Amount of cadaverine in crystal produced, g** | 147 | 173 | 185 | 195 | 184 | 172 | 158 |
| **Amount of cadaverine in mother liquor, g** | 490 | 448 | 430 | 414 | 431 | 449 | 477 |

| **Results** | | | | | | | |
|---|---|---|---|---|---|---|---|
| **Purity, %** | 99.0 | 99.0 | 99.0 | 99.0 | 99.0 | 99.0 | 88.7 |
| **Yield, %** | 23.0 | 27.9 | 30.0 | 32.0 | 29.9 | 27.8 | 24.8 |
| **Total yield,** % | 41.6 | 48.5 | 51.3 | 53.8 | 51.0 | 48.2 | 43.4 |

Table 3 is for the process liquids to which the mother liquor was circulated two times.

**[Table 3]**

| **Two times of mother liquor circulation** | **Exampl e 1-1** | **Exampl e 1-2** | **Exampl e 1-3** | **Exampl e 1-4** | **Exampl e 1-5** | **Exampl e 1-6** | **Exampl e 1-7** |
|---|---|---|---|---|---|---|---|
| **Molar ratio of succinate /cadaverine** | 0.8 | 0.9 | 0.95 | 1.01 | 1.05 | 1.1 | 1.2 |

| **Feed liquid for crystallization** | | | | | | | |
|---|---|---|---|---|---|---|---|
| **Fresh feed liquid, ml** | 1000 | 1000 | 1000 | 1000 | 1000 | 1000 | 1000 |
| **Amount of cadaverine in crystallization liquid, g** | 852 | 810 | 792 | 776 | 793 | 811 | 839 |
| **Amount of cadaverine in crystal produced, g** | 186 | 221 | 235 | 248 | 234 | 219 | 209 |
| **Amount of cadaverine in mother liquor, g** | 666 | 589 | 557 | 527 | 559 | 591 | 630 |

| **Results** | | | | | | | |
|---|---|---|---|---|---|---|---|
| **Purity, %** | 99.0 | 99.0 | 99.0 | 99.0 | 99.0 | 99.0 | 88.9 |
| **Yield, %** | 21.9 | 27.2 | 29.7 | 32.0 | 29.5 | 27.1 | 24.9 |
| **Total yield,** % | 54.4 | 62.5 | 65.7 | 68.6 | 65.5 | 62.2 | 57.5 |

Table 4 is for the process liquids to which the mother liquor was circulated three times.

**[Table 4]**

| **Three times of mother liquor circulation** | **Exampl e 1-1** | **Exampl e 1-2** | **Exampl e 1-3** | **Exampl e 1-4** | **Exampl e 1-5** | **Exampl e 1-6** | **Exampl e 1-7** |
|---|---|---|---|---|---|---|---|
| **Molar ratio of succinate /cadaverine** | 0.8 | 0.9 | 0.95 | 1.01 | 1.05 | 1.1 | 1.2 |

| **Feed liquid for crystallization** | | | | | | | |
|---|---|---|---|---|---|---|---|
| **Fresh feed liquid, ml** | 1000 | 1000 | 1000 | 1000 | 1000 | 1000 | 1000 |
| **Amount of cadaverine in crystallization liquid, g** | 1028 | 951 | 919 | 889 | 921 | 953 | 992 |
| **Amount of cadaverine in crystal produced, g** | 212 | 252 | 269 | 285 | 268 | 251 | 247 |
| **Amount of cadaverine in mother liquor, g** | 816 | 699 | 649 | 605 | 654 | 703 | 745 |

| **Results** | | | | | | | |
|---|---|---|---|---|---|---|---|
| **Purity, %** | 99.0 | 99.0 | 99.0 | 99.0 | 99.0 | 98.6 | 89.0 |
| **Yield, %** | 20.6 | 26.5 | 29.3 | 32.0 | 29.0 | 26.3 | 24.9 |
| **Total yield, %** | 63.8 | 72.4 | 75.8 | 78.6 | 75.5 | 72.2 | 68.1 |

Table 5 is for the process liquids to which the mother liquor was circulated four times.

**[Table 5]**

| **Four times of mother liquor circulation** | **Example 1-1** | **Example 1-2** | **Example 1-3** | **Example 1-4** | **Example 1-5** | **Example 1-6** | **Example 1-7** |
|---|---|---|---|---|---|---|---|
| **Molar ratio of succinate /cadaverine** | 0.8 | 0.9 | 0.95 | 1.01 | 1.05 | 1.1 | 1.2 |
| **Feed liquid for crystallization** | | | | | | | |
| **Fresh feed liquid, ml** | 1000 | 1000 | 1000 | 1000 | 1000 | 1000 | 1000 |
| **Amount of cadaverine in crystallization liquid, g** | 1178 | 1061 | 1011 | 967 | 1016 | 1065 | 1107 |
| **Amount of cadaverine in crystal produced, g** | 227 | 272 | 292 | 309 | 290 | 274 | 276 |
| **Amount of cadaverine in mother liquor, g** | 950 | 789 | 720 | 657 | 726 | 791 | 831 |

| **Results** | | | | | | | |
|---|---|---|---|---|---|---|---|
| **Purity, %** | 99.0 | 99.0 | 99.0 | 99.0 | 99.0 | 98.1 | 89.0 |
| **Yield, %** | 19.3 | 25.7 | 28.8 | 32.0 | 28.5 | 25.7 | 24.9 |
| **Total yield, %** | 70.8 | 79.5 | 82.8 | 85.5 | 82.5 | 79.3 | 76.0 |

Summarizing the experimental results according to Tables 1 to 5 above, it has been found that the preparation of cadaverine succinate overall having an improved crystallization rate, a high purity, and a high yield is possible through the preparation method of the present disclosure in which diammonium succinate in the first step is contained in a large amount to be a level of 81 to 100 mol% of the total succinate supplied.

More specifically, Example 1-1 has a high purity of 99% but has a low yield of 70.8% when the mother liquor is circulated four times. This is because the sequentially circulated mother liquor lowered the molar ratio of succinate in the feed liquid for crystallization and the concentration of succinate was insufficient compared to the concentration of cadaverine succinate.

Example 1-2 has a high purity of 99% but has a slightly low yield of 79.5% when the mother liquor is circulated four times. This is because the sequentially circulated mother liquor lowered the molar ratio of succinate in the feed liquid for crystallization and the concentration of succinate was insufficient compared to the concentration of cadaverine succinate.

On the other hand, Example 1-3 has a high purity of 99% and a high total yield of 82.8% when the mother liquor is circulated four times.

Example 1-4 has a high purity of 99% and a high total yield of 85.5% when the mother liquor is circulated four times.

Example 1-5 has a high purity of 99% and a high total yield of 82.5% when the mother liquor is circulated four times.

Example 1-6 has a slightly low purity of 98.1% and a slightly low yield of 79.3% when the mother liquor is circulated four times. This is because succinic acid contained in an amount exceeding the solubility was precipitated as crystals and the purity was thus decreased.

Example 1-7 has a low purity of 89% and a low yield of 76% when the mother liquor is circulated four times. This is because succinic acid contained in an amount exceeding the solubility was precipitated as crystals and the purity was thus decreased.

As described above, it has been revealed that a method for preparing highly pure cadaverine succinate can be provided depending on the molar ratio of succinate added throughout the process to the cadaverine in the process liquid after the second step. In particular, it has been found that when the amount of succinate added with respect to the amount of cadaverine in the process liquid after the second step is adjusted to about 0.95 to 1.05 in terms of molar ratio, an excellent total yield of 82% or more can be achieved while a purity of cadaverine succinate of 99% is maintained when the mother liquor is circulated four or more times as well.

### Preparation Example 2. Preparation of cadaverine glutarate

### Preparation Example 2-1. Lysine glutarate production and fermentation process

Seeds of *Corynebacterium glutamicum* strain (KCCM12154P, US 2021-0355514 A1) having the ability to produce lysine were obtained through solid phase culture and flask culture, seed culture was performed in a fermenter, and then the main production and fermentation were performed. Fermentation was carried out through fermenter culture at 36°C for 30 hours at 900 rpm.

For the production of lysine glutarate using *Corynebacterium,* diammonium glutarate was supplied at a level of 66 to 100 g/L to replace the existing ammonium sulfate, and fermentation was performed to obtain lysine glutarate at a level of 111 to 167 g/L and lysine at a level of 92 g/L. At this time, in Examples, the molar ratio of glutarate /lysine in the fermentation process liquid was adjusted to a level of 0.63 to 0.95.

### Preparation Example 2-2. Conversion reaction of lysine glutarate into cadaverine glutarate

For the conversion reaction, *Escherichia coli* (US 2018-0030430 A1) in which PtLDC enzyme, a lysine decarboxylase gene derived from *Pseudomonas thermotolerans,* was overexpressed with the pET-Deut1 vector was used.

The enzyme conversion liquid obtained by seed culture with the enzyme strain was supplied to the fermentation process liquid of lysine glutarate prepared in Preparation Example 2-1 at a level of 10% by mass to conduct the conversion reaction. The conversion reaction was conducted for approximately 1 hour while the temperature was maintained at 45°C to 50°C. The pH was adjusted to 8.0 to 8.5, and for this, CO₂ was introduced for neutralization in Example 2-1 to 2-4 below and a dicarboxylic acid was additionally introduced at a level of 0.01 to 0.05 mol/L (based on the volume of the fermentation process liquid of lysine glutarate) in Examples 2-1 to 2-3 and 2-4. As a result, it was found that the conversion rate into cadaverine glutarate was 97% to 98%. Specifically, after the conversion reaction, cadaverine succinate was obtained at a level of 101 to 141 g/L (based on the volume of the fermentation process liquid of lysine glutarate) and cadaverine was obtained at a level of 63 g/L (based on the volume of the fermentation process liquid of lysine glutarate). Subsequently, in Examples 2-4 to 2-7, glutaric acid was additionally introduced at a level of 0.02 to 0.08 mol/L to prepare a process liquid.

### Preparation Example 2-3. Subsequent processes after obtaining of cadaverine glutarate

The following subsequent processes were performed on the process liquid containing cadaverine glutarate obtained in Preparation Example 2-2 to obtain cadaverine glutarate in a crystal form.

### [Bacteria removing step]

Bacteria in the cadaverine glutarate process liquid were removed by performing membrane filtration using a membrane having a size of 0.1 µm.

### [Step of removing impurities using activated carbon]

Activated carbon was added at a level of 10% based on the weight of cadaverine glutarate in the filtrate from which the bacteria had been separated. The process liquid to which activated carbon had been added was heated to 60°C and stirred for 1 hour to decolorize, and then the activated carbon was filtered through filter paper.

### [Step of concentrating cadaverine glutarate process liquid from which impurities had been removed]

The liquid filtered above was concentrated under reduced pressure in a rotary evaporator at about 45°C to 65°C and 40 to 70 torr until the solid content in the filtrate became 75% to 82% by weight.

### [Steps of cooling and crystallizing, separating and then drying cadaverine glutarate concentrate]

The concentrate was cooled from 50°C to 35°C to 25°C at a rate of 5°C/hr. The precipitated crystals were separated from the mother liquor using a centrifuge. The crystals were washed with 10% water based on the weight of cadaverine glutarate. The separated crystals were dried for one day, and then the purity thereof was measured by HPLC.

### [Step of recirculating mother liquor to crystallization step]

The mother liquor separated above was recirculated to the [step of concentrating cadaverine glutarate process liquid from which impurities had been removed].

### Experimental Example 2. Examination of changes in purity and yield of cadaverine glutarate depending on molar ratio of glutarate to cadaverine

In Experimental Example 2, the method according to Preparation Examples 2-1 to 2-3 was used, but it was examined how the purity and yield of cadaverine glutarate change depending on the molar ratio of glutarate added throughout the process to cadaverine in the process liquid after the second step.

### Example 2-1: Case where molar ratio of glutarate added/cadaverine in process liquid after second step is 0.7

A liquid for conversion into cadaverine glutarate having a molar ratio of glutarate /cadaverine of 0.7 was supplied by 1,000 ml and allowed to pass through a membrane having a size of 0.1 µm to remove microorganisms. The liquid for conversion into cadaverine glutarate having a molar ratio of glutarate /cadaverine of 0.7 was prepared according to thepreparation method of Preparation Example 2-1 and Preparation Example 2-2 but the sum of the amount of diammonium glutarate introduced in the fermentation process to produce lysine in Preparation Example 2-1. and the amount of glutaric acid introduced in the reaction for conversion into cadaverine glutarate (limited to Examples in which glutaric acid was additionally introduced in the second step) in Preparation Example 2-2. was controlled to be 0.7 of the number of moles of cadaverine that had been converted.

The filtrate was decolorized using activated carbon, filtered through filter paper, then concentrated under reduced pressure at 40 torr, and concentrated so that the solid content became a level of 75% by weight. The concentrate was cooled and crystallized from 50°C to 25°C. The crystals and mother liquor were separated from each other through centrifugation, the separated crystals were dried for one day, and the purity thereof was then measured by HPLC. The mother liquor in which a glutarate remained was recirculated to the cadaverine glutarate feed liquid having a molar ratio of glutarate /cadaverine of 0.7, and then the concentration step was performed again. Recirculation of the mother liquor was carried out sequentially.

### Example 2-2: Case where molar ratio of glutarate added/cadaverine in process liquid after second step is 0.79

A liquid for conversion into cadaverine glutarate having a molar ratio of glutarate /cadaverine of 0.79 was supplied, and the purification process was performed while the mother liquor was sequentially recirculated in the same manner as in Example 2-1.

### Example 2-3: Case where molar ratio of glutarate added/cadaverine in process liquid after second step is 0.89

A liquid for conversion into cadaverine glutarate having a molar ratio of glutarate /cadaverine of 0.89 was supplied, and the purification process was performed while the mother liquor was sequentially recirculated in the same manner as in Example 2-1.

### Example 2-4: Case where molar ratio of glutarate added/cadaverine in process liquid after second step is 0.95

A liquid for conversion into cadaverine glutarate having a molar ratio of glutarate /cadaverine of 0.95 was supplied, and the purification process was performed while the mother liquor was sequentially recirculated in the same manner as in Example 2-1.

### Example 2-5: Case where molar ratio of glutarate added/cadaverine in process liquid after second step is 1.0

A liquid for conversion into cadaverine glutarate having a molar ratio of glutarate /cadaverine of 1.0 was supplied, and the purification process was performed while the mother liquor was sequentially recirculated in the same manner as in Example 2-1.

In Examples 2-3 to 2-5, the molar ratio of glutariate/lysine in the lysine fermentation step was adjusted to be 0.79 to 0.95.

### Example 2-6: Case where molar ratio of glutarate added/cadaverine in process liquid after second step is 1.05

A liquid for conversion into cadaverine glutarate having a molar ratio of glutarate /cadaverine of 1.05 was supplied, and the purification process was performed while the mother liquor was sequentially recirculated in the same manner as in Example 2-1.

### Example 2-7: Case where molar ratio of glutarate added/cadaverine in process liquid after second step is 1.10

A liquid for conversion into cadaverine glutarate having a molar ratio of glutarate /cadaverine of 1.10 was supplied, and the purification process was performed while the mother liquor was sequentially recirculated in the same manner as in Example 2-1.

The measured physical properties of the process liquids and cadaverine glutarate prepared according to Examples 2-1 to 2-7 described above are as shown in Tables 6 to 9 below.

Table 6 is for the process liquids to which the mother liquor is not circulated.

**[Table 6]**

| **No mother liquor circulation** | **Example 2-1** | **Example 2-2** | **Example 2-3** | **Example 2-4** | **Example 2-5** | **Example 2-6** | **Example 2-7** |
|---|---|---|---|---|---|---|---|
| **Molar ratio of glutarate /cadaverine** | 0.70 | 0.79 | 0.89 | 0.95 | 1.00 | 1.05 | 1.10 |

| **Feed liquid for crystallization** | | | | | | | |
|---|---|---|---|---|---|---|---|
| **Fresh feed liquid, ml** | 1000 | 1000 | 1000 | 1000 | 1000 | 1000 | 1000 |
| **Amount of cadaverine in crystallization liquid, g** | 327 | 327 | 327 | 327 | 327 | 327 | 327 |
| **Amount of cadaverine in crystal produced, g** | 141 | 153 | 162 | 166 | 170 | 166 | 161 |
| **Amount of cadaverine in mother liquor, g** | 186 | 174 | 165 | 161 | 157 | 161 | 166 |

| **Results** | | | | | | | |
|---|---|---|---|---|---|---|---|
| **Purity, %** | 99.0 | 99.0 | 99.0 | 99.0 | 99.0 | 95.0 | 90.0 |
| **Yield, %** | 43.0 | 46.8 | 49.7 | 50.9 | 52.0 | 50.6 | 49.3 |
| **Total yield, %** | 43.0 | 46.8 | 49.7 | 50.9 | 52.0 | 50.6 | 49.3 |

Table 7 is for the process liquids to which the mother liquor is circulated one time.

**[Table 7]**

| **One time of mother liquor circulation** | **Exampl e 2-1** | **Exampl e 2-2** | **Exampl e 2-3** | **Exampl e 2-4** | **Exampl e 2-5** | **Exampl e 2-6** | **Exampl e 2-7** |
|---|---|---|---|---|---|---|---|
| **Molar ratio of glutarate /cadaverine** | 0.70 | 0.79 | 0.89 | 0.95 | 1.00 | 1.05 | 1.10 |

| **Feed liquid for crystallization** | | | | | | | |
|---|---|---|---|---|---|---|---|
| **Fresh feed liquid, ml** | 1000 | 1000 | 1000 | 1000 | 1000 | 1000 | 1000 |
| **Amount of cadaverine in crystallization liquid, g** | 513 | 501 | 492 | 488 | 484 | 488 | 493 |
| **Amount of cadaverine in crystal produced, g** | 208 | 226 | 240 | 246 | 252 | 248 | 246 |
| **Amount of cadaverine in mother liquor, g** | 305 | 275 | 251 | 241 | 232 | 240 | 247 |

| **Results** | | | | | | | |
|---|---|---|---|---|---|---|---|
| **Purity, %** | 99.0 | 99.0 | 99.0 | 99.0 | 99.0 | 95.3 | 90.8 |
| **Yield, %** | 40.6 | 45.2 | 48.9 | 50.5 | 52.0 | 50.9 | 50.0 |
| **Total yield, %** | 66.1 | 70.8 | 74.3 | 75.7 | 77.0 | 75.8 | 74.6 |

Table 8 is for the process liquids to which the mother liquor is circulated two times.

**[Table 8]**

| **Two times of mother liquor circulation** | **Example 2-1** | **Example 2-2** | **Example 2-3** | **Example 2-4** | **Example 2-5** | **Example 2-6** | **Example 2-7** |
|---|---|---|---|---|---|---|---|
| **Molar ratio of glutarate /cadaverine** | 0.70 | 0.79 | 0.89 | 0.95 | 1.00 | 1.05 | 1.10 |

| **Feed liquid for crystallization** | | | | | | | |
|---|---|---|---|---|---|---|---|
| **Fresh feed liquid, ml** | 1000 | 1000 | 1000 | 1000 | 1000 | 1000 | 1000 |
| **Amount of cadaverine in crystallization liquid, g** | 632 | 602 | 578 | 568 | 559 | 567 | 574 |
| **Amount of cadaverine in crystal produced, g** | 238 | 260 | 277 | 284 | 291 | 289 | 289 |
| **Amount of cadaverine in mother liquor, g** | 394 | 342 | 301 | 284 | 268 | 278 | 284 |

| **Results** | | | | | | | |
|---|---|---|---|---|---|---|---|
| **Purity, %** | 99.0 | 99.0 | 99.0 | 99.0 | 99.0 | 95.4 | 91.3 |
| **Yield, %** | 37.7 | 43.2 | 47.9 | 50.0 | 52.0 | 51.0 | 50.4 |
| **Total yield, %** | 78.9 | 83.4 | 86.6 | 87.9 | 88.9 | 88.1 | 87.4 |

Table 9 is for the process liquids to which the mother liquor is circulated three times.

**[Table 9]**

| **Three times of mother liquor circulation** | **Example 2-1** | **Example 2-2** | **Example 2-3** | **Example 2-4** | **Example 2-5** | **Example 2-6** | **Example 2-7** |
|---|---|---|---|---|---|---|---|
| **Molar ratio of glutarate /cadaverine** | 0.70 | 0.79 | 0.89 | 0.95 | 1.00 | 1.05 | 1.10 |

| **Feed liquid for crystallization** | | | | | | | |
|---|---|---|---|---|---|---|---|
| **Fresh feed liquid, ml** | 1000 | 1000 | 1000 | 1000 | 1000 | 1000 | 1000 |
| **Amount of cadaverine in crystallization liquid, g** | 721 | 669 | 628 | 611 | 595 | 605 | 611 |
| **Amount of cadaverine in crystal produced, g** | 248 | 273 | 293 | 302 | 310 | 309 | 310 |
| **Amount of cadaverine in mother liquor, g** | 473 | 396 | 335 | 309 | 286 | 296 | 301 |

| **Results** | | | | | | | |
|---|---|---|---|---|---|---|---|
| **Purity, %** | 99.0 | 99.0 | 99.0 | 99.0 | 99.0 | 95.5 | 91.6 |
| **Yield, %** | 34.3 | 40.7 | 46.6 | 49.4 | 52.0 | 51.1 | 50.7 |
| **Total yield, %** | 86.1 | 90.2 | 92.8 | 93.9 | 94.7 | 94.2 | 93.8 |

Summarizing the experimental results according to Tables 6 to 9 above, it has been found that the preparation of cadaverine glutarate overall having an improved crystallization rate, a high purity, and a high yield is possible through the preparation method of the present disclosure in which diammonium glutarate in the first step is contained in a large amount to be a level of 88 to 100 mol% of the total glutariate supplied.

More specifically, Example 2-1 has a high purity of 99% but has a low yield of 86.1% when the mother liquor is circulated three times. This is because the sequentially circulated mother liquor lowered the molar ratio of glutarate in the feed liquid for crystallization and the concentration of glutarate was insufficient compared to the concentration of cadaverine glutarate.

Example 2-2 has a high purity of 99% and a high yield of 90.2% when the mother liquor is circulated three times.

Meanwhile, Example 2-3 has a high purity of 99% and a high yield of 92.8% when the mother liquor is circulated three times.

Example 2-4 has a high purity of 99% and a high total yield of 93.9% when the mother liquor is circulated three times.

Example 2-5 has a high purity of 99% and a high total yield of 94.7% when the mother liquor is circulated three times.

Example 2-6 has a low purity of 95.5% and a yield of 94.2% when the mother liquor is circulated three times. This is because glutaric acid contained in an amount exceeding the solubility was precipitated as crystals and the purity was thus decreased, and the concentration of glutarate was insufficient compared to the concentration of cadaverine glutarate.

Example 2-7 has a lower purity compared to Example 2-5, and having a purity of 91.6% and a slightly low yield of 93.8% when the mother liquor is circulated three times. This is because glutaric acid contained in an amount exceeding the solubility was precipitated as crystals and the purity was thus decreased, and the concentration of glutarate was insufficient compared to the concentration of cadaverine glutarate.

As described above, it has been revealed that a method for preparing highly pure cadaverine glutarate can be provided depending on the molar ratio of glutarate added throughout the process to the cadaverine in the process liquid after the second step. In particular, it has been found that when the amount of glutarate added with respect to the amount of cadaverine in the process liquid after the second step is adjusted to about 0.89 to 1.0 in terms of molar ratio, an excellent total yield of 92% or more can be achieved while a purity of cadaverine glutarate of 99% is maintained when the mother liquor is circulated three or more times as well.

From the above description, those skilled in the art to which the present disclosure belongs will be able to understand that the present disclosure can be implemented in other specific forms without changing its technical idea or essential features. Therefore, it should be understood that the embodiments described above are not limitative but illustrative in all aspects. The scope of the present disclosure is defined by the appended claims rather than by the description preceding them, and all changes and modifications derived from the meaning and scope of the claims or equivalents thereof should be construed as being included in the scope of the present disclosure.

## Claims

1. A method for preparing cadaverine dicarboxylate comprising:
a first step of obtaining a lysine dicarboxylate by culturing an L-lysine producing microorganism in a medium containing a dicarboxylate in a form of a diammonium dicarboxylate; and
a second step of converting the lysine dicarboxylate into a cadaverine dicarboxylate, wherein
an amount of dicarboxylate added, with respect to an amount of cadaverine in a process liquid after the second step, is 0.85 to 1.07 in terms of molar ratio, and
the dicarboxylate is an aliphatic dicarboxylate having 4 or 5 carbon atoms.

2. The method according to claim 1, wherein a diammonium dicarboxylate in the first step is contained to be 81 mol% or more of the total dicarboxylate supplied.

3. The method according to claim 1, wherein a diammonium dicarboxylate in the first step is contained so that a molar ratio of the diammonium dicarboxylate to lysine to be produced (diammonium dicarboxylate/lysine) is 0.78 to 0.98.

4. The method according to claim 1, wherein a conversion reaction in the second step is conducted at a pH of 8.0 to 8.5.

5. The method according to claim 1, wherein the dicarboxylate is succinate, and
an amount of dicarboxylate added with respect to an amount of cadaverine in a process liquid after the second step is 0.95 to 1.05 in terms of molar ratio.

6. The method according to claim 1, wherein the dicarboxylate is glutarate, and
an amount of dicarboxylate added with respect to an amount of cadaverine in a process liquid after the second step is 0.89 to 1.0 in terms of molar ratio.

7. The method according to claim 1, wherein the second step comprises additionally adding a dicarboxylic acid to a process liquid.

8. The method according to claim 1, further comprising a recovery step of recovering a cadaverine dicarboxylate after the second step.

9. The method according to claim 7, wherein the recovery step comprises at least one or more steps selected from the group consisting of a filtration step, a concentration step, and a cooling crystallization step.

10. The method according to claim 7, wherein the recovery step further comprises a mother liquor circulation step of recirculating a mother liquor containing a cadaverine dicarboxylate that has not been crystallized to a feed liquid for a concentration step after the cooling crystallization step.

11. The method according to claim 8, wherein the concentration step is to perform concentration so that a solid content becomes 75% to 82% (w/w).

12. The method according to claim 1, wherein conversion of a lysine dicarboxylate into a cadaverine dicarboxylate in the second step is performed using a protein having lysine decarboxylase activity or a microorganism expressing a protein having the activity.

13. The method according to claim 1, wherein the L-lysine producing microorganism is *Corynebacterium glutamicum.*

14. The method according to claim 1, wherein the recovery step does not comprise a separate process for removing a dicarboxylate in a process liquid.

15. The method according to claim 9, wherein the dicarboxylate is succinate, and
a cadaverine dicarboxylate having a purity of 99% or more is obtained in 0 to 4 times of mother liquor circulation.

16. The method according to claim 9, wherein the dicarboxylate is glutarate, and
a cadaverine dicarboxylate having a purity of 99% or more is obtained in 0 to 3 times of mother liquor circulation.
